# EUROPÄISCHE PATENTSCHRIFT

(11) **EP 0 628 541 B1**
(45) Veröffentlichungstag und Bekanntmachung des Hinweises auf die Patenterteilung: **16.04.1997**
(21) Anmeldenummer: 94108216.6
(22) Anmeldetag: 28.05.1994
(51) Int. Cl.: C07C 267/00, C07C 275/40, C07C 271/20

(54) **Carbodiimide und/oder oligomere Polycarbodiimide auf Basis von 1,3-Bis-(1-methyl-1-isocyanatoethyl)-benzol, ein Verfahren zu ihrer Herstellung und ihre Verwendung als Hydrolysestabilisator**
Carbodiimides and/or oligomeric polycarbodiimides based on 1,3-bis-(1-methyl-1-isocyanatoethyl)-benzene, process for their preparation and their use as stabilizer against hydrolysis
Carbodiimides et/ou polycarbodiimides oligomérique à base de 1,3-bis-(1-méthyle-1-isocyanatoéthyl)-benzène, procédé de leur préparation et leur utilisation comme stabilisateurs d'hydrolyse

(30) Priorität: 08.06.1993 DE 4318979
(43) Veröffentlichungstag der Anmeldung: 14.12.1994
(73) Patentinhaber: BASF Aktiengesellschaft, 67063 Ludwigshafen (DE)
(72) Erfinder: Pohl, Siegmund, D-67069 Ludwigshafen (DE); Lehrich, Friedhelm, Dr., D-49448 Lemfoerde (DE); Genz, Manfred, Dr., D-49401 Damme (DE); Bruchmann, Bernd, Dr., D-67069 Ludwigshafen (DE); Tesch, Helmut, Dr., D-67127 Roedersheim-Gronau (DE); Minges, Roland, Dr., D-67269 Gruenstadt (DE); Streu, Joachim, Dr., D-85221 Dachau (DE)

(56) Entgegenhaltungen:
- EP-A- 0 460 481
- US-A- 2 941 983
- PATENT ABSTRACTS OF JAPAN, vol. 5, no. 195 (C-83)(867), 11. Dezember 1981 & JP-A-56 118 053

## Beschreibung

Gegenstände der Erfindung sind neue Carbodiimide oder oligomere Polycarbodiimide mit endständigen Isocyanat-, Harnstoff- und/oder Urethangruppen der Formel (I) 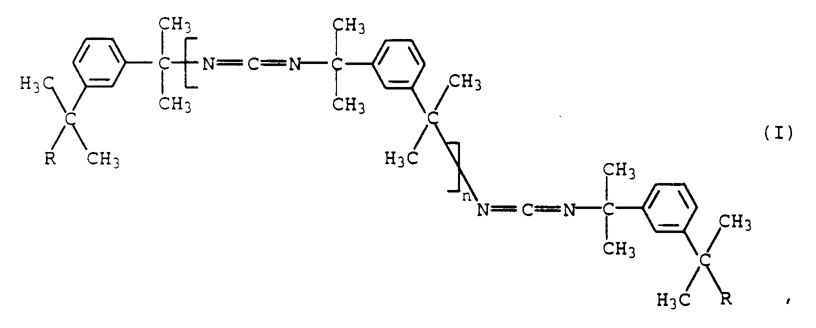 in der R gleich oder verschieden und ausgewählt ist aus der Gruppe der -NCO-, -NHCONHR¹-, -NHCONR¹R²- und -NHCOOR³-Reste und n eine ganze Zahl von 0 bis 10 bedeutet, ein Verfahren zu ihrer Herstellung und ihre Verwendung als Stabilisator gegen den hydrolytischen Abbau von Estergruppen enthaltenden Polyadditions- und Polykondensationsprodukten, vorzugsweise von Polyurethanen.

Organische Carbodiimide sind bekannt. Ihre Chemie wird z.B. beschrieben in Chemical Reviews, Vol. 53 (1953), Seiten 145 bis 166 und Angewandte Chemie 74 (1962), Seiten 801 bis 806.

Monocarbodiimide und oligomere Polycarbodiimide können beispielsweise hergestellt werden durch Einwirkung von basischen Katalysatoren auf sterisch gehinderte Mono- oder Polyisocyanate unter Kohlendioxidabspaltung. Als basische Katalysatoren geeignet sind z.B. gemäß GB-A-1 083 410 heterocyclische, Phosphor gebunden enthaltende Verbindungen und nach Angaben der DE-B-1 130 594 (GB-A-851 936) Phospholene und Phospholidine sowie deren Oxide und Sulfide.

Beschrieben werden ferner Polycarbodiimide mit endständigen Urethangruppen z.B. in der US-A-2 941 983 und DE-B-22 48 751 (US-A-4 076 945). Die Produkte können beispielsweise hergestellt werden durch Carbodiimidisierung von Diisocyanaten mit sterisch gehinderten Isocyanatgruppen und anschließende partielle oder vollständige Urethanisierung der endständigen NCO-Gruppen mit Alkoholen. Bei Verwendung von aromatischen Diisocyanaten mit Isocyanatgruppen unterschiedlicher Reaktivität können die Isocyanatgruppen mit höherer Reaktivität zunächst mit Alkohol partiell oder vollständig in die entsprechenden Urethangruppen übergeführt und danach die verbliebenen Isocyanatgruppen unter Kohlendioxidabspaltung zu Carbodiimidgruppen umgesetzt werden. Oligomere Polycarbodiimide mit einem mittleren Kondensationsgrad von 2 bis 30, die erhältlich sind durch Oligokondensation von 2,4'-Diisocyanato-diphenylmethan oder eines 3,3',5,5'-Tetra-C₁- bis -C₄-alkyl-4,4'-diisocyanato-diphenylmethans oder von Mischungen dieser gegebenenfalls alkylsubstituierten Diisocyanato-diphenylmethane mit weiteren zwei- oder mehrfunktionellen aromatischen Isocyanaten und gewünschtenfalls vollständige oder teilweise Umsetzung der noch freien Isocyanatgruppen der erhaltenen oligomeren Polycarbodiimide mit einem aliphatischen, araliphatischen oder cycloaliphatischen Alkohol oder Amin werden in der DE-A-41 26 359 beschrieben.

Die Carbodiimide finden vorzugsweise Verwendung als Stabilisatoren gegen die hydrolytische Spaltung von Kunststoffen auf Polyesterbasis. Nach Angaben der DE-A-1 494 009 (US-A-3 193 523) eignen sich hierfür insbesondere aromatische und/oder cycloaliphatische Monocarbodiimide, die in 2- und 2'-Stellung substituiert sind, wie z.B. 2,2',6',6'-Tetraisopropyl-diphenylcarbodiimid. Polycarbodiimide mit einem Molekulargewicht von über 500 und einem Gehalt von mehr als 3 Carbodiimidgruppen werden als Stabilisatoren gegen Einflüsse von Wärme und Feuchtigkeit in estergruppenhaltigen Kunststoffen in der DE-B-1 285 747 (US-A-3 193 522) beschrieben. Obgleich durch den Zusatz dieser (Poly)carbodiimide als Stabilisatoren eine weitgehende Stabilität Estergruppen haltiger Kunststoffe gegen feuchte Wärme, Wasser und Wasserdampf erzielt werden kann, weisen die Produkte auch Nachteile auf. Nachteilig an den technisch vorzugsweise verwendeten tetra-alkylsubstituierten Monocarbodiimiden, wie z.B. 2,2',6,6'-Tetraisopropyldiphenyl-carbodiimid, ist ihr relativ hoher Dampfdruck und bedingt durch das niedrige Molekulargewicht ihre Neigung zur Migration aus den Polyadditionsprodukten, z.B. thermoplastischen Polyurethanen (TPU), oder Polykondensationsprodukten, z.B. Polyterephthalaten. Zur Beseitigung diese Mangels finden nach Angaben der EP-A-0 460 481 (CA-A-2 043 820) substituierte Monocarbodiimide oder oligomere, substituierte Polycarbodiimide mit endständigen Isocyanatgruppen Verwendung, die hergestellt werden aus substituierten Diisocyanaten und die praktisch weder in der Hitze, z.B. unter den üblichen Verarbeitungsbedingungen, noch bei Raumtemperatur giftige, flüchtige aus den eingesetzten Carbodiimiden stammende Substanzen abspalten. Polycarbodiimide dieser Art besitzen höhere Schmelzpunkte oder sind unschmelzbar und können nur mit einem beträchtlichen apparativen und zeitlichen Aufwand in die Polyurethane und/oder ihre Ausgangsstoffe eingebracht werden. Die Verteilung der Polycarbodiimide in den Estergruppen enthaltenden Kunststoffen ist daher vielfach unzureichend homogen, so daß die Stabilisatorwirksamkeit nicht den Erwartungen entspricht. Durch die Überführung eines Teils der endständigen Isocyanat- in Urethangruppen, z.B. gemäß DE-A-22 48 751 oder US-A-2 941 983, können leichter schmelzende Polycarbodiimidderivate erhalten werden.

Die Aufgabe der vorliegenden Erfindung bestand darin, die vorgenannten Nachteile ganz oder zumindest teilweise zu beseitigen und Hydrolyseschutzmittel zur Verfügung zu stellen, die homogen in den Estergruppen enthaltenden Kunststoffen, vorzugsweise Polyurethanen und insbesondere TPU, löslich sind, ohne daß es hierzu zusätzlicher Homogenisierungsschritte bedarf. Die Hydrolyseschutzmittel sollten insbesondere ein toxikologisch verbessertes Verhalten aufweisen.

Diese Aufgabe konnte überraschenderweise gelöst werden durch die Verwendung von 1,3-Bis-(1-methyl-1-isocyanato-ethyl)-benzol zur Herstellung von Carbodiimiden und/oder oligomeren Polycarbodiimiden mit endständigen Isocyanat-, Harnstoff- und/oder Urethangruppen.

Gegenstand der Erfindung sind somit Carbodiimide und/oder oligomere Polycarbodiimide der Formel (I) 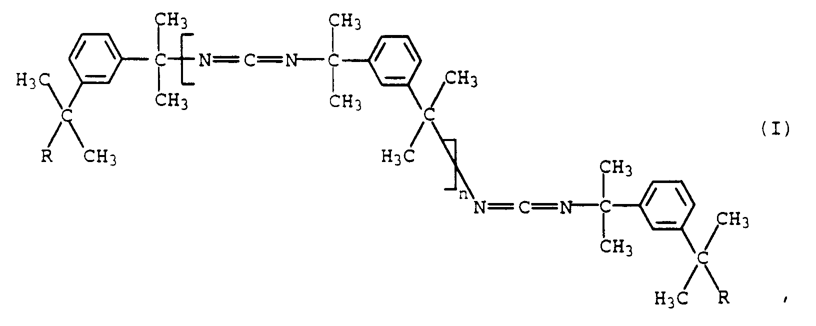 in der
- R: gleich oder verschieden und ausgewählt ist aus der Gruppe der -NCO-, -NHCONHR¹-, -NHCONR¹R²- und -NHCOOR³-Reste,
wobei R¹ und R² gleich oder verschieden sind und einen Alkyl-, Cycloalkyl- oder Aralkylrest bedeuten und
R³ gleich R¹ oder ein Alkoxypolyoxyalkylenrest ist und
n eine ganze Zahl von 0 bis 10 ist.

Gegenstände der Erfindung sind ferner Verfahren zur Herstellung der erfindungsgemäßen, neuen Carbodiimide und/oder oligomeren Polycarbodiimide der Formeln (I) und (II) gemäß den Ansprüchen 6 oder 7 und die Verwendung der Carbodiimide und/oder oligomeren Polycarbodiimide der Formeln (I) und (II) als Stabilisatoren gegen den hydrolytischen Abbau von Estergruppen gebunden enthaltenden Polyadditions- oder Polykondensationsprodukte gemäß Anspruch 9.

Die erfindungsgemäßen Carbodiimide und oligomeren Polycarbodiimide besitzen an eine Methylengruppe gebundene, sterisch gehinderte Isocyanat-, Harnstoff- und/oder Urethangruppen, zeigen eine mit den technisch genutzten aromatischen Carbodiimiden und aromatischen Polycarbodiimiden zumindest vergleichbare hohe Hydrolyseschutzwirkung bei erhöhter Lichtbeständigkeit und können unter Beachtung der Arbeitsschutzvorschriften problemlos ohne zusätzliche Homogenisierungsschritte wirtschaftlich dosiert und in die Estergruppen enthaltenden Polykondensations- und Polyadditionsprodukte eingebracht werden. Vorteilhaft ist ferner die große Anzahl an wirksamen Carbodiimidgruppen, bezogen auf das Molekulargewicht der (Poly)carbodiimide, ihr geringer Dampfdruck und das vernachlässigbare Migrations- und Ausblühverhalten. Die (Poly)carbodiimide sind mit den Estergruppen enthaltenden Polyadditions-und Polykondensationsprodukten, insbesondere mit Polyesterurethankautschuken, gut verträglich und aufgrund ihres niedrigen Schmelzpunktes auch problemlos mit diesen Materialien in der Schmelze homogen mischbar.

Aus den erfindungsgemäßen Carbodiimiden und oligomeren Polycarbodiimiden entstehen bei der Reaktion mit Carbonsäuren und/oder carboxylgruppenhaltigen Verbindungen araliphatische Isocyanate mit einer im Vergleich zu aromatischen Isocyanaten geringen Reaktivität. Die gebildeten araliphatischen Isocyanate beeinflussen daher die Kennzahl der Polyadditionsreaktion bei der Urethanbildung praktisch nicht. Folglich sind die Molekulargewichte der gebildeten Polyurethane und damit verbunden ihre mechanischen Eigenschaften konstant und sehr gut reproduzierbar. Vorteilhaft ist ferner, daß die aus den Isocyanaten entstehenden Abbauprodukte keine aromatischen Amingruppen gebunden haben und daher toxikologisch als relativ unproblematisch anzusehen sind.

Neben den monomeren Carbodiimiden finden als oligomere Polycarbodiimide vorteilhafterweise solche mit einem mittleren Kondensationsgrad (Zahlenmittelwert) von 2 bis 10, vorzugsweise von 2 bis 5 oder Mischungen davon oder Mischungen aus Mono- und oligomeren Polycarbodiimiden Verwendung, da diese sich in der Regel besonders gut in die zu stabilisierenden Estergruppen enthaltenden Polyadditions- oder Polykondensationsprodukte einbringen lassen. Höherkondensierte Polycarbodiimide sind in der Regel feste, hochschmelzende Verbindungen, die mit der Kunststoffmatrix unzureichend verträglich und daher schwieriger mit den Polyadditions- oder Polykondensationsprodukten homogen mischbar sind.

Die erfindungsgemäßen Carbodiimide und oligomeren Polycarbodiimide der Formel (II) 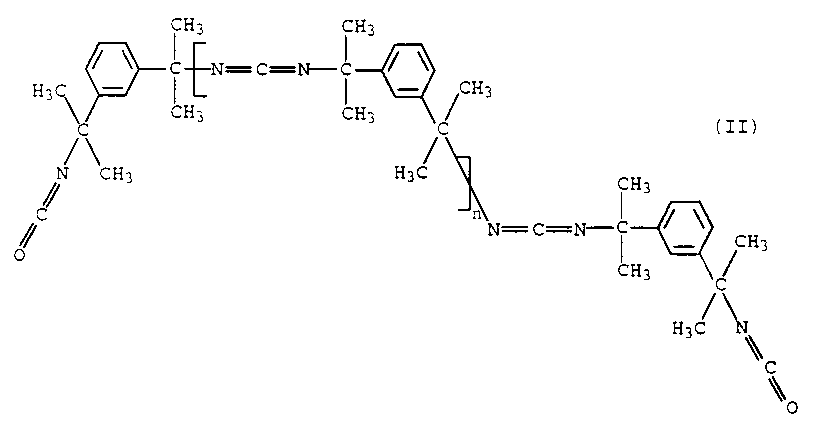 besitzen noch reaktive Isocyanatgruppen und können daher mit Verbindungen mit NCO-reaktiven Wasserstoffatomen reagieren und auf diese Weise in den Polyadditions- oder Polykondensationsprodukten chemisch gebunden werden. Zur Verbesserung der Lagerbeständigkeit der (Poly)carbodiimide können die endständigen Isocyanatgruppen beispielsweise mit C-H- oder N-H-reaktiven Verbindungen, wie z.B. Malonester, Acetylaceton, Acetessigester, Phthalimid, Caprolactam oder Benzolsulfonamid ganz oder teilweise verkappt oder durch Umsetzung mit aliphatischen, cycloaliphatischen oder araliphatischen Aminen, Alkoholen oder Polyoxyalkylenalkoholen teilweise oder vollständig abgesättigt und dadurch ihre physikalischen Eigenschaften, z.B. ihre Löslichkeit oder Verträglichkeit, gezielt modifiziert werden.

Zur Absättigung der Isocyanatgruppen der (Poly)carbodiimide können, wie bereits ausgeführt wurde, Amine, Alkohole und Polyoxyalkylenalkohole, verwendet werden. Geeignete Amine, z.B. primäre oder vorzugsweise sekundäre Amine, besitzen vorteilhafterweise 1 bis 12 C-Atome, vorzugsweise 2 bis 8 C-Atome. Beispielhaft genannt seien Methyl-, Ethyl-, Propyl-, Butyl-, Pentyl-, Hexyl-, 2-Ethylhexyl-, Octyl-, Decyl-, Diethyl-, Dipropyl-, Dibutyl-, Methylbutyl-, Ethylbutyl- und Ethylhexylamin sowie Cyclohexyl- und Benzylamin. Zur Absättigung der Isocyanatgruppen finden jedoch vorzugsweise Alkohole, z.B. primäre oder sekundäre Alkohole mit 1 bis 18 C-Atomen, vorzugsweise 2 bis 8 C-Atomen und insbesondere Alkoxypolyoxyalkylenalkohole mit 1 bis 10 C-Atomen, vorzugsweise 1 bis 4 C-Atomen, in der Alkoxygruppe und einem Molekulargewicht von 76 bis 2000, vorzugsweise 400 bis 1000 (Zahlenmittel) Verwendung. Als primäre oder sekundäre Alkohole seien beispielhaft genannt: Methanol, Ethanol, n-Propanol, Isopropanol, n-Butanol, sekundär-Butanol, n-Pentanol, technische Pentanolmischungen, n-Hexanol, technische Hexanolgemische, 2-Ethylhexanol, Octanol, 2-Ethyloctanol, Decanol und Dodecanol sowie Cyclohexanol und Benzylalkohol. Als Alkoxypolyoxyalkylenalkohole haben sich beispielsweise Polyoxybutylen-, Polyoxypropylen-, Polyoxypropylen-polyoxyethylen- und vorzugsweise Polyoxyethylenalkohole bewährt, die als endständige Alkoxygruppe, beispielsweise eine Methoxy-, Ethoxy-, n- oder iso-Propoxy- oder n-Butoxygruppe gebunden haben können. Je nach Art der verwendeten Polyoxyalkylenreste können die (Poly)carbodiimide hydrophil, wasserlöslich, bis hydrophob, fettlöslich, eingestellt werden.

Zur Herstellung der erfindungsgemäßen Carbodiimide und/oder oligomeren Polycarbodiimide kann das 1,3-Bis-(1-methyl-1-isocyanato-ethyl)-benzol bei erhöhten Temperaturen, d.h. bei Temperaturen von 50 bis 200°C, vorzugsweise von 150 bis 185°C, in Gegenwart von Katalysatoren unter Kohlendioxidabspaltung kondensiert werden. Hierfür geeignete Verfahren werden beispielsweise beschrieben in der GB-A-1 083 410, der DE-B-1 130 594 (GB-A-851 936) und der DE-A-11 56 401 (US-A-3 502 722). Als Katalysatoren vorzüglich bewährt haben sich z.B. Phosphorverbindungen, die vorzugsweise ausgewählt werden aus der Gruppe der Phospholene, Phospholenoxide, Phospholidine und Phospholinoxide. Wenn die Reaktionsmischung den gewünschten Gehalt an NCO-Gruppen, entsprechend einem Kondensationsgrad n bis 10 besitzt, wird die Polycarbodiimidbildung üblicherweise beendet. Hierzu können die Katalysatoren unter vermindertem Druck abdestilliert oder durch Zusatz eines Desaktivators, wie z.B. Phosphortrichlorid, desaktiviert werden. Die Polycarbodiimidherstellung kann ferner in Abwesenheit oder Gegenwart von unter den Reaktionsbedingungen inerten Lösungsmitteln durchgeführt werden.

Durch geeignete Wahl der Reaktionsbedingungen wie z.B. der Reaktionstemperatur, der Katalysatorart und der Katalysatormenge sowie der Reaktionszeit kann der Fachmann in der üblichen Weise den Kondensationsgrad einstellen. Der Verlauf der Reaktion kann am einfachsten durch Bestimmung des NCO-Gehaltes verfolgt werden. Auch andere Parameter wie z.B. Viskositätsanstieg, Farbvertiefung oder CO₂-Entwicklung kann man für den Ablauf und die Steuerung der Reaktion heranziehen.

Nach beendeter Kondensation können, wie bereits ausgeführt wurde, die freien endständigen Isocyanatgruppen des Carbodiimids und/oder der oligomeren Polycarbodiimide mit C-H- oder N-H-reaktiven Wasserstoffverbindungen blockiert oder mit aliphatischen, cycloaliphatischen und/oder araliphatischen Aminen, derartigen Alkoholen und/oder Alkoxypolyoxyalkylenalkoholen vollständig oder teilweise abgesättigt werden. Nach einer vorteilhaften Ausführungsform werden zur vollständigen Absättigung der Isocyanatgruppen die aliphatischen, cycloaliphatischen oder araliphatischen Amine, Alkohole und/oder Alkoxypolyoxyalkylenalkohole vorzugsweise in einem geringen Überschuß von -OH-, -NH- und/oder -NH₂-Gruppen zu NCO-Gruppen der (Poly)carbodiimide enthaltenden Reaktionsmischung hinzugefügt, dort ausreagieren gelassen und danach gegebenenfalls die nicht umgesetzte Menge, bevorzugt unter vermindertem Druck abdestilliert.

Nach einer anderen, vorzugsweise angewandten Verfahrensvariante können die erfindungsgemäßen (Poly)carbodiimide mit teilweise oder vollständig abgesättigten Isocyanatgruppen in der Weise hergestellt werden, daß man zunächst bis zu 50 Gew.-%, vorzugsweise bis zu 23 Gew.-% der Isocyanatgruppen des 1,3-Bis-(1-methyl-1-isocyanato-ethyl)-benzols mit mindestens einem aliphatischen, cycloaliphatischen oder araliphatischen Amin, Alkohol und/oder Alkoxypolyoxyalkylenalkohol umsetzt und danach die freien Isocyanatgruppen in Gegenwart von Katalysatoren unter Kohlendioxidabspaltung ganz oder teilweise zu Carbodiimiden und/oder oligomeren Polycarbodiimiden kondensiert.

Die erfindungsgemäßen Monocarbodiimide und/oder oligomeren Polycarbodiimide eignen sich hervorragend als Akzeptor für Carboxylverbindungen und finden daher vorzugsweise Verwendung als Stabilisatoren gegen den hydrolytischen Abbau von Estergruppen gebunden enthaltende Polykondensationsprodukte z.B. Polyester, Polyetherester, Polyesteramide und Polycaprolactone, und Polyadditionsprodukte, z.B. Polyurethane, Polyharnstoffe und PolyurethanPolyharnstoff-Elastomere. Aufgrund der guten Löslichkeit, insbesondere der Monocarbodiimide, in den Aufbaukomponenten zur Herstellung von Polyurethanen und der guten Verträglichkeit, insbesondere der oligomeren Polycarbodiimide, mit den gebildeten Polyurethanen sowie der Reaktivität der NCO-Gruppen enthaltenden (Poly)carbodiimide mit Verbindungen mit reaktiven Wasserstoffatomen eignen sich die erfindungsgemäßen (Poly)carbodiimide insbesondere als Stabilisatoren gegen den hydrolytischen Abbau von Polyurethanen, vorzugsweise kompakten oder zelligen Polyurethan-Elastomeren und insbesondere TPU.

Die Konzentration der erfindungsgemäßen Monocarbodiimide und/oder oligomeren Polycarbodiimide in den zu stabilisierenden Estergruppen enthaltenden Polykondensations- oder Polyadditionsprodukte beträgt im allgemeinen 0,05 bis 10 Gew.-%, vorzugsweise 0,1 bis 5 Gew.-%. In Einzelfällen kann, je nach der Beanspruchung des Kunststoffs durch Hydrolyse, die Konzentration auch höher sein.

Die erfindungsgemäß verwendbaren Monocarbodiimide und/oder oligomeren Polycarbodiimide können nach verschiedenen Methoden in die zu stabilisierende Estergruppen enthaltende Polyadditions- oder Polykondensationsprodukte eingebracht werden. Beispielsweise können die erfindungsgemäßen (Poly)carbodiimide mit einer der Aufbaukomponenten zur Herstellung der Polyadditionsprodukte, z.B. den Polyisocyanaten oder/und Polyhydroxylverbindungen zur Herstellung von Polyurethanen gemischt werden oder die (Poly)carbodiimide können der Reaktionsmischung zur Herstellung der Polyurethane zudosiert werden. Nach einer anderen Verfahrensweise können die erfindungsgemäßen (Poly)carbodiimide der Schmelze der ausreagierten Polyadditions- oder Polykondensationsprodukte einverleibt werden. Es ist jedoch auch möglich Granulate der Polyadditions- oder Polykondensationsprodukte mit den erfindungsgemäßen (Poly)carbodiimiden zu beschichten und diese bei einer nachfolgenden Herstellung von Formkörpern durch Schmelzextrusion in die Kunststoffmassen einzubringen. Zur Herstellung von Polyurethan-Gießelastomeren und TPU auf Polyester-polyolbasis werden nach einer bevorzugten Ausführungsform zunächst die carboxylgruppenhaltigen Polyester-polyole zur Reduzierung der Säuregehalte mit den erfindungsgemäßen (Poly)carbodiimiden behandelt und danach diese, gegebenenfalls unter Zugabe von weiteren Mengen an (Poly)carbodiimiden, mit Polyisocyanaten, gegebenenfalls in Gegenwart zusätzlicher Hilfsmittel und Additive, zur Reaktion gebracht.

Neben der Wirksamkeit als Stabilisator gegen den hydrolytischen Abbau von Estergruppen enthaltenden Polyadditions- oder Polykondensationsprodukten oder zur Entsäuerung von Polyesterolen, welche zur Herstellung von polyesterhaltigen Kunststoffen, insbesondere Polyurethankautschuken, verwendet werden können, eignen sich die Monocarbodiimide und/oder oligomeren Polycarbodiimide z.B. auch zum Abbruch von Veresterungsreaktionen bei der Herstellung von Polyestern, wenn der gewünschte Polykondensationsgrad erreicht ist.

### Beispiele

### Beispiel 1

750 Gew.-Teile (3,1 mol) 1,3-Bis-(1-methyl-1-isocyanato-ethyl)-benzol mit einem NCO-Gehalt von 34,4 Gew.-% wurden in Gegenwart von 1,5 Gew.-Teilen 1-Methyl-2-phospholen-1-oxid lösungsmittelfrei auf 180°C erhitzt und bei dieser Temperatur unter mäßiger Kohlendioxidentwicklung kondensiert. Nach Erreichen eines NCO-Gehalts der Reaktionsmischung von 5,2 Gew.-%, hierzu war eine Reaktionszeit von ungefähr 22 Stunden erforderlich, wurde der zugesetzte Katalysator und Reste von nicht umgesetztem 1,3-Bis-(1-methyl-1-isocyanato-ethyl)-benzols bei einer Temperatur von 190°C und unter einem Druck von 0,2 mbar abdestilliert.

Man erhielt 549 Gew. -Teile einer Mischung aus Mono- und oligomeren Polycarbodiimiden mit einem NCO-Gehalt von 4,5 Gew.-%, einem Gehalt an -N=C=N-Gruppen von 141 mg/g, einem Schmelzpunkt < 30°C und einer Jodfarbzahl von 4,0, gemessen nach DIN 6162.

Die Struktur der Isocyanatgruppen aufweisenden Mischung aus Mono- und oligomeren Polycarbodiimiden wurde durch ¹H-NMR- und IR-Spektrum nachgewiesen.

### Beispiel 2

750 Gew.-Teile (3,1 mol) 1,3-Bis-(1-methyl-1-isocyanato-ethyl)-benzol mit einem NCO-Gehalt von 34,4 Gew.-% wurden in Gegenwart von 1,5 Gew.-Teilen 1-Methyl-2-phospholen-1-oxid lösungsmittelfrei auf 180°C erhitzt und bei dieser Temperatur unter Kohlendioxidentwicklung kondensiert. Nach Erreichen eines NCO-Gehalts von 5 Gew.-%, hierzu war eine Reaktionszeit von ungefähr 23 Stunden erforderlich, wurden unter Rühren 495 Gew.-Teile eines Methoxypolyoxyethylenalkohols mit einem durchschnittlichen Molekulargewicht (Zahlenmittelwert) von 520 g/mol hinzugefügt.

Bei Einhaltung der Reaktionstemperatur von 180°C wurden die endständigen NCO-Gruppen in einem Zeitraum von 30 Minuten in Urethangruppen übergeführt.

Danach wurden das als Katalysator zugesetzte 1-Methyl-2-phospholen-1-oxid und Reste von nicht umgesetztem 1,3-Bis-(1-methyl-1-isocyanato-ethyl)-benzol bei einer Temperatur von 190°C und unter einem Druck von 0,5 mbar abdestilliert.

Man erhielt 1049 Gew.-Teile einer NCO-Gruppen freien Mischung aus endständige Methoxypolyoxyethylen-Urethangruppen aufweisenden Mono- und oligomeren Polycarbodiimiden, die einen Gehalt an -N=C=N-Gruppen von 76 mg/g, eine Viskosität, gemessen bei 25°C nach Ubbelohde von 15620 mPa·s und eine Jodfarbzahl, gemessen verdünnt mit Monochlorbenzol im Volumenverhältnis von 1:5, von 3,8 besaß. Die Mischung war in Wasser wenig löslich.

Die Struktur der Urethangruppen aufweisenden Mischung aus Mono- und oligomeren Polycarbodiimiden wurde durch ¹H-NMR- und IR-Spektrum nachgewiesen.

### Beispiel 3

Man verfuhr analog den Angaben des Beispiels 2, kondensierte das 1,3-Bis-(1-methyl-1-isocyanato-ethyl)-benzol jedoch bis auf einen NCO-Gehalt von 9,3 Gew.-%.

Die NCO-Gruppen enthaltende Mischung aus Mono- und oligomeren Polycarbodiimiden wurde danach durch Umsetzung mit 957 Gew-.Teilen eines Methoxypolyoxyethylenalkohols mit einem durchschnittlichen Molekulargewicht von 520 (Zahlenmittelwert) analog den Angaben des Beispiels 2 urethanisiert und destilliert.

Man erhielt 1576 Gew.-Teile einer NCO-Gruppen freien Mischung aus endständige Methoxypolyoxyethylen-Urethangruppen aufweisenden Mono- und oligomeren Polycarbodiimiden, die einen Gehalt an -N=C=N-Gruppen von 50 mg/g, eine Viskosität, gemessen bei 25°C nach Ubbelohde von 1950 mPa·s und eine Jodfarbzahl, gemessen verdünnt mit Monochlorbenzol im Volumenverhältnis 1:5 von 2,4 besaß. Die Mischung war in Wasser von 23°C sehr gut löslich.

Die Struktur der Urethangruppen aufweisenden Mischung aus Mono- und oligomeren Polycarbodiimiden wurde durch ¹H-NMR- und IR-Spektrum nachgewiesen.

### Beispiel 4

Man verfuhr analog den Angaben des Beispiels 2, kondensierte das 1,3-Bis-(1-methyl-1-isocyanato-ethyl)-benzol jedoch bis auf einen NCO-Gehalt von 7,9 Gew.-%.

Die NCO-Gruppen enthaltende Mischung aus Mono- und oligomeren Polycarbodiimiden wurde danach zur teilweisen Umsetzung der NCO-Gruppen mit 183 Gew.-Teilen 2-Ethylhexanol-1 analog den Angaben des Beispiels 2 urethanisiert und destilliert.

Die erhaltene Mischung von 843 Gew.-Teilen aus endständige 2-Ethyhlhexyl-Urethangruppen aufweisenden Mono- und oligomeren Polycarbodiimiden hatte noch einen NCO-Gehalt von 1,8 % und besaß einen Gehalt an -N=C=N-Gruppen von 94 mg/g, einen Schmelzpunkt < 30°C und eine Jodfarbzahl, gemessen verdünnt mit Monochlorbenzol im Volumenverhältnis 1:5, von 1,5.

Die Struktur der Urethangruppen aufweisenden Mischung aus Mono- und oligomeren Polycarbodiimiden wurde durch ¹H-NMR und IR-Spektrum nachgewiesen.

Herstellung von mit den erfindungsgemäßen Mischungen aus Mono- und oligomeren Polycarbodiimiden stabilisierten thermoplastischen Polyurethanen

Aus einem thermoplastischen Polyurethan-Granulat, das durch Umsetzung von
- 440 g: (1,76 mol) 4,4'-Diphenylurethan-diisocyanat,
- 1000 g: (0,5 mol) 1,4-Butandiol-1,6-Hexandiol-Polyadipat und
- 113 g: (1,26 mol) 1,4-Butandiol
hergestellt wurde und als Hydrolysestabilisator Carbodiimide und/oder oligomere Polycarbodiimide enthielt, wurden mit Hilfe des Spritzgußverfahrens Prüfkörper hergestellt.

An den Prüfkörpern wurde unmittelbar nach ihrer Herstellung und nach einer 21tägigen Lagerung bei 80°C in Wasser die Zugfestigkeit nach DIN 53 504 und die Bruchdehnung nach DIN 53 504 gemessen.

Die verwendeten Carbodiimide und/oder oligomeren Polycarbodiimide, ihre Mengen und die gemessenen mechanischen Eigenschaften sind in der Tabelle zusammengefaßt.

**Tabelle**

| Vergleichsbeispiel | Carbodiimid und/oder oligomere Polycarbodiimide hergestellt | | Zugfestigkeit nach | | Bruchdehnung nach | |
|---|---|---|---|---|---|---|
| | Art | Menge [Gew.-%, bezogen auf TPU] | 0 [MPa] | 21 Tagen [MPa] | 0 [%] | 21 Tagen [%] |
| I | - | - | 51 | 2 | 590 | 75 |
| II | aus 2,6-Diisopropyl-phenyl-isocyanat | 0,65 | 59 | 37 | 620 | 680 |
| III | aus Triisopropyl-phenyl-isocyanat | 0,65 | 54 | 40 | 610 | 700 |

| Beispiel | | | | | | |
|---|---|---|---|---|---|---|
| 5 | nach Beispiel 1 | 0,65 | 60 | 39 | 630 | 780 |
| 6 | nach Beispiel 1 | 1,00 | 60 | 38 | 670 | 750 |
| 7 | nach Beispiel 1 | 1,30 | 57 | 35 | 670 | 750 |
| 8 | nach Beispiel 2 | 0,65 | 52 | 36 | 580 | 520 |
| 9 | nach Beispiel 4 | 0,65 | 56 | 37 | 560 | 530 |

## Patentansprüche

1. Carbodiimide und/oder oligomere Polycarbodiimide der Formel (I) 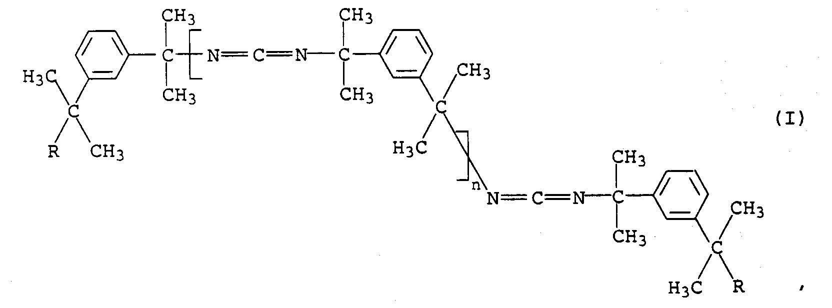 in der
R gleich oder verschieden und ausgewählt ist aus der Gruppe der -NCO-, -NHCONHR¹-, -NHCONR¹R²- und -NHCOOR³-Reste, wobei
R¹ und R² gleich oder verschieden sind und einen Alkyl-, Cycloalkyl- oder Aralkylrest bedeuten und
R³ gleich R¹ oder ein Alkoxypolyoxyalkylenrest ist und
n eine ganze Zahl von 0 bis 10 ist.

2. Carbodiimide und/oder oligomere Polycarbodiimide der Formel (I), in der R eine -NHCOOR³-Gruppe ist und R³ einen Alkoxypolyoxyethylenrest mit einem Molekulargewicht von 76 bis 2000 und n eine ganze Zahl von 0 bis 10 bedeuten.

3. Carbodiimid und/oder oligomere Polycarbodiimide der Formel (II) 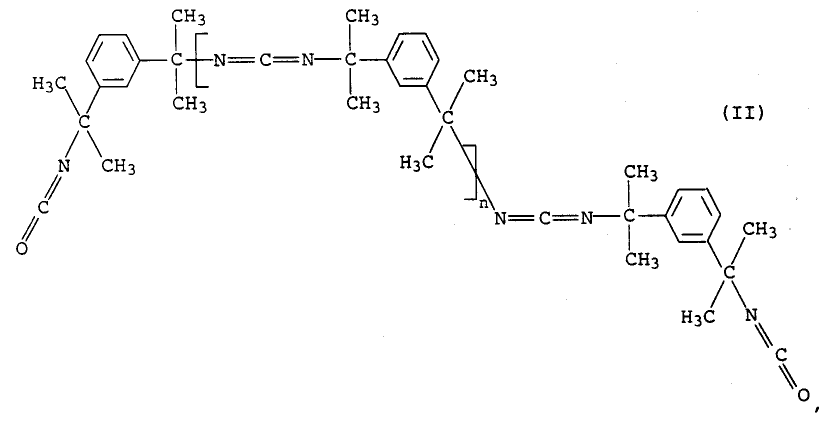 in der n eine ganze Zahl von 0 bis 10 ist.

4. Carbodiimide und/oder oligomere Polycarbodiimide der Formel (I), erhältlich
a) durch Kondensation in Gegenwart von Katalysatoren unter Kohlendioxidabspaltung bei Temperaturen von 50 bis 200°C von 1,3-Bis-(1-methyl-1-isocyanato-ethyl)-benzol und gegebenenfalls teilweise oder vollständige Umsetzung der endständigen Isocyanatgruppen des erhaltenen Carbodiimids oder/und der erhaltenen oligomeren Polycarbodiimide mit mindestens einem aliphatischen, cycloaliphatischen oder araliphatischen Amin, Alkohol und/oder Alkoxypolyoxyalkylenalkohol oder
b) durch katalytische Umsetzung von bis zu 50 % der Isocyanatgruppen des 1,3-Bis-(1-methyl-1-isocyanato-ethyl)-benzols mit mindestens einem aliphatischen, cycloaliphatischen oder araliphatischen Amin, Alkohol und/oder Alkoxypolyoxyalkylenalkohol und anschließende Kondensation der freien Isocyanatgruppen unter Kohlendioxidabspaltung.

5. Carbodiimide und/oder oligomere Polycarbodiimide der Formel (II), erhältlich durch Kondensation unter Kohlendioxidabspaltung von 1,3-Bis-(1-methyl-1-isocyanato-ethyl)-benzol.

6. Verfahren zur Herstellung von Carbodiimiden und/oder oligomeren Polycarbodiimiden der Formel (I), dadurch gekennzeichnet, daß man
a) 1,3-Bis-(1-methyl-isocyanato-ethyl)-benzol in Gegenwart von Katalysatoren unter Kohlendioxidabspaltung bei Temperaturen von 50 bis 200°C kondensiert, anschließend gegebenenfalls die freien endständigen Isocyanatgruppen der erhaltenen (Poly)carbodiimide teilweise oder vollständig mit mindestens einem aliphatischen, cycloaliphatischen oder araliphatischen Amin, Alkohol und/oder Alkoxypolyoxyalkylenalkohol umsetzt und danach die Katalysatoren abtrennt oder desaktiviert
oder
b) bis zu 50 % der Isocyanatgruppen des 1,3-Bis-(1-methyl-1-isocyanato-ethyl)-benzols mit mindestens einem aliphatischen, cycloaliphatischen oder araliphatischen Amin, Alkohol und/oder Alkoxypolyoxyalkylenalkohol umsetzt, anschließend die freien Isocyanatgruppen in Gegenwart von Katalysatoren unter Kohlendioxidabspaltung ganz oder teilweise kondensiert und danach die Katalysatoren abtrennt oder desaktiviert.

7. Verfahren zur Herstellung eines Carbodiimids und/oder von oligomeren Polycarbodiimiden der Formel (II), dadurch gekennzeichnet, daß man 1,3-Bis-(1-methyl-1-isocyanato-ethyl)-benzol in Gegenwart von Katalysatoren unter Kohlendioxidabspaltung kondensiert und danach die Katalysatoren abtrennt oder desaktiviert.

8. Verfahren nach Anspruch 6 oder 7, dadurch gekennzeichnet, daß man als Katalysator zur Kondensation mindestens eine Phosphorverbindung, ausgewählt aus der Gruppe der Phospholene, Phospholenoxide, Phospholidine und Phospholidinoxide verwendet.

9. Verwendung von Carbodiimiden und/oder oligomeren Polycarbodiimiden der Formel (I) als Stabilisator gegen den hydrolytischen Abbau von Estergruppen enthaltenden Polyadditions- oder Polykondensationsprodukten.

10. Verwendung von Carbodiimiden und/oder oligomeren Polycarbodiimiden der Formel (I) als Stabilisator gegen den hydrolytischen Abbau von Polyurethanen.

## Claims

1. A carbodiimide or oligomeric polycarbodiimide of the formula (I) 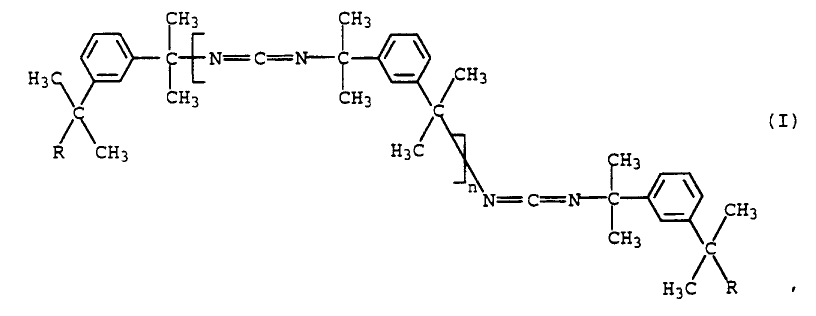 where
the R radicals are identical or different and are selected from the group consisting of -NCO-, -NHCONHR¹-, -NHCONR¹R²- and -NHCOOR³-, where
R¹ and R² may be identical or different and are alkyl, cycloalkyl or aralkyl, and
R³ is identical to R¹ or is alkoxypolyoxyalkylene, and
n is an integer from 0 to 10.

2. A carbodiimide or oligomeric polycarbodiimide of the formula (I), where R is -NHCOOR³-, and R³ is alkoxypolyoxyethylene having a molecular weight of from 76 to 2000, and n is an integer from 0 to 10.

3. A carbodiimide or oligomeric polycarbodiimide of the formula (II) 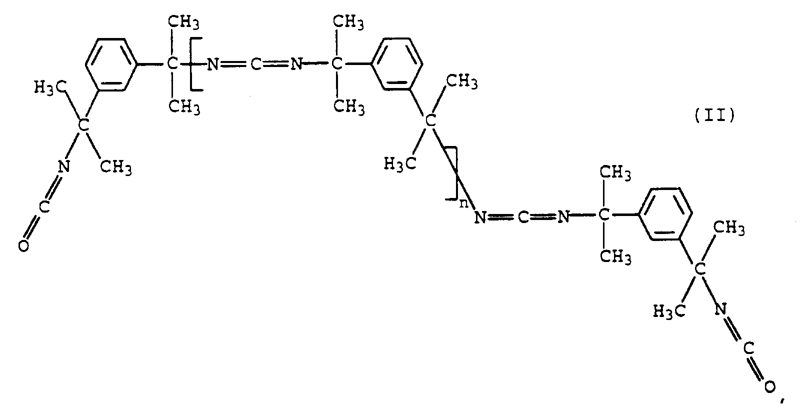 where n is an integer from 0 to 10.

4. A carbodiimide or oligomeric polycarbodiimide of the formula (I), obtainable
a) by condensation of 1,3-bis(1-methyl-1-isocyanatoethyl)benzene at from 50 to 200°C in the presence of catalysts with elimination of carbon dioxide, and, if necessary, reaction of all or some of the terminal isocyanate groups of the resultant carbodiimide or oligomeric polycarbodiimide with at least one aliphatic, cycloaliphatic or araliphatic amine, alcohol and/or alkoxypolyoxyalkylene alcohol, or
b) by catalytic reaction of up to 50% of the isocyanate groups of 1,3-bis(1-methyl-1-isocyanatoethyl)benzene with at least one aliphatic, cycloaliphatic or araliphatic amine, alcohol and/or alkoxypolyoxyalkylene alcohol, and subsequent condensation of the free isocyanate groups with elimination of carbon dioxide.

5. A carbodiimide or oligomeric polycarbodiimide of the formula (II), obtainable by condensation of 1,3-bis-(1-methyl-1-isocyanatoethyl)benzene with elimination of carbon dioxide.

6. A process for the preparation of a carbodiimide or oligomeric polycarbodiimide of the formula (I), which comprises
a) condensing 1,3-bis(1-methylisocyanatoethyl)benzene at from 50 to 200°C in the presence of catalysts with elimination of carbon dioxide, if necessary subsequently reacting all or some of the free terminal isocyanate groups of the resultant (poly)carbodiimide with at least one aliphatic, cycloaliphatic or araliphatic amine, alcohol and/or alkoxypolyoxyalkylene alcohol, and then removing or deactivating the catalysts,
or
b) reacting up to 50% of the isocyanate groups of 1,3-bis(1-methyl-1-isocyanatoethyl)benzene with at least one aliphatic, cycloaliphatic or araliphatic amine, alcohol and/or alkoxypolyoxyalkylene alcohol, subsequently condensing all or some of the free isocyanate groups in the presence of catalysts with elimination of carbon dioxide, and then removing or deactivating the catalysts.

7. A process for the preparation of a carbodiimide or oligomeric polycarbodiimide of the formula (II), which comprises condensing 1,3-bis(1-methyl-1-isocyanatoethyl)-benzene in the presence of catalysts with elimination of carbon dioxide, and then removing or deactivating the catalysts.

8. A process as claimed in claim 6 or 7, wherein the catalyst used for the condensation is at least one phosphorus compound selected from the group consisting of phospholenes, phospholene oxides, phospholidines and phospholidine oxides.

9. The use of a carbodiimide or oligomeric polycarbodiimide of the formula (I) as a stabilizer against hydrolytic degradation of polyaddition or polycondensation products containing ester groups.

10. The use of a carbodiimide or oligomeric polycarbodiimide of the formula (I) as a stabilizer against hydrolytic degradation of polyurethanes.

## Revendications

1. Carbodiimides et/ou polycarbodiimides oligomériques de la formule (I) 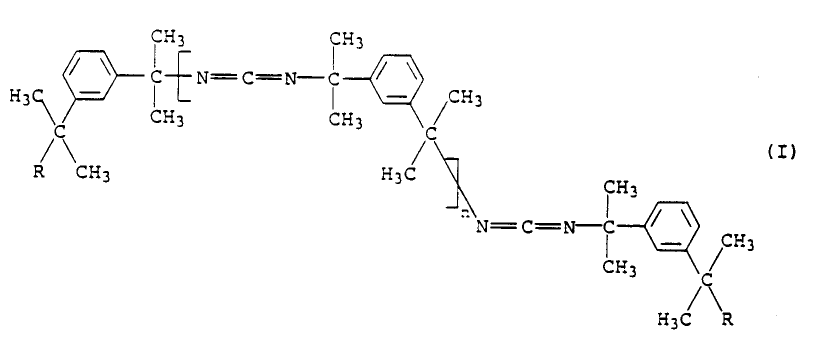 dans laquelle
les symboles R sont identiques ou différents et choisis dans le groupe formé par les radicaux -NCO-, -NHCONHR¹-, -NHCONR¹R²- et -NHCOOR³, où
R¹ et R² sont identiques ou différents et représentent chacun un groupe alkyle, cycloalkyle ou aralkyle et
R³ est identique à R¹ ou représente un groupe alcoxypolyoxyalkylène et
n représente un nombre entier dont la valeur varie de 0 à 10.

2. Carbodiimides et/ou polycarbodiimides oligomériques de la formule (I), dans laquelle R représente un groupe -NHCOOR³ et R³ représente un groupe alcoxypolyoxyéthylène, d'un poids moléculaire de 76 à 2000 et n représente un nombre entier dont la valeur varie de 0 à 10.

3. Carbodiimides et/ou polycarbodiimides oligomériques, répondant à la formule (II) 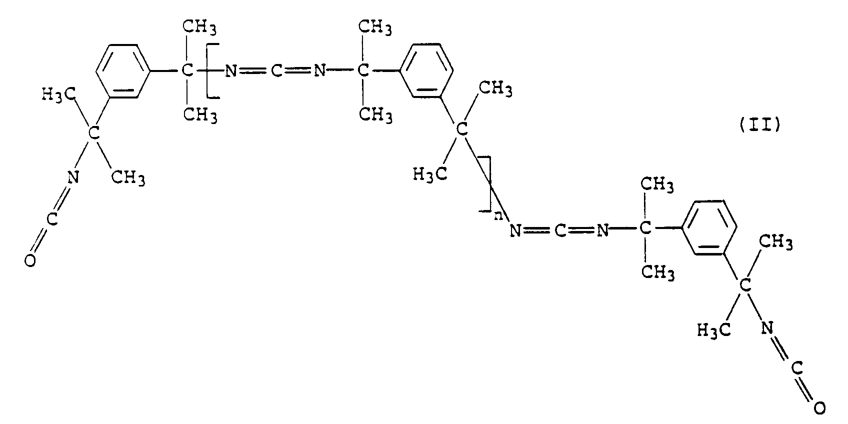 dans laquelle n représente un nombre entier dont la valeur varie de 0 à 10.

4. Carbodiimides et/ou polycarbodiimides oligomériques, répondant à la formule (I), que l'on peut obtenir
a) par la condensation, en présence de catalyseurs et sous séparation de dioxyde de carbone, à des températures de 50 à 200°C, du 1,3-bis(1-méthyl-1-isocyanato-éthyl)benzène et éventuellement la réaction partielle ou totale des groupes isocyanate terminaux du carbodiimide obtenu et/ou des polycarbodiimides oligomériques obtenus, avec au moins un alcoxypolyoxyalkylènealcool, un alcool et/ou une amine, aliphatique, cycloaliphatique ou araliphatique, ou bien
b) par la réaction catalytique de jusqu'à 50% des radicaux isocyanate du 1,3-bis(1-méthyl-1-isocyanato-éthyl)benzène, avec au moins un alcoxypolyoxyalkylènealcool, un alcool et/ou une amine, aliphatique, cycloaliphatique ou araliphatique et la condensation subséquente des radicaux isocyanate libres, sous Séparation de dioxyde de carbone.

5. Carbodiimides et/ou polycarbodiimides oligomériques, répondant à la formule (II) que l'on peut obtenir par la condensation sous séparation de dioxyde de carbone du 1,3-bis(1-méthyl-1-isocyanato-éthyl)benzène.

6. Procédé de préparation de carbodiimides et/ou de polycarbodiimides oligomériques, répondant à la formule (I), caractérisé en ce que
a) on condense le 1,3-bis(1-méthyl-1-isocyanatoéthyl)benzène, en présence de catalyseurs, sous séparation de dioxyde de carbone, à des températures de 50 à 200°C, on fait ensuite éventuellement réagir les radicaux isocyanate terminaux libres des (poly)carbodiimides obtenus, partiellement ou totalement, avec au moins un alcoxypolyoxyalkylènealcool, un alcool et/ou une amine, aliphatique, cycloaliphatique ou araliphatique et on sépare ou désactive ensuite les catalyseurs, ou bien
b) on fait réagir jusqu'à 50% des radicaux isocyanate du 1,3-bis(1-méthyl-1-isocyanato-éthyl)benzène, avec au moins un alcoxypolyoxyalkylènealcool, un alcool et/ou une amine, aliphatique, cycloaliphatique, ou araliphatique, on condense ensuite, totalement ou partiellement, les radicaux isocyanate libres, en présence de catalyseurs et sous séparation de dioxyde de carbone et on sépare ou désactive finalement les catalyseurs.

7. Procédé de préparation d'un carbodiimide et/ou d'un polycarbodiimide oligomérique, répondant à la formule (II), caractérisé en ce que l'on condense le 1,3-bis(1-méthyl-1-isocyanato-éthyl)benzène, en présence de catalyseurs, sous séparation de dioxyde de carbone et on sépare ou désactive ensuite les catalyseurs.

8. Procédé suivant la revendication 6 ou 7, caractérisé en ce que l'on choisit, à titre de catalyseur, pour la condensation, au moins un composé du phosphore, appartenant au groupe des phospholènes, des oxydes de phospholène, des phospholidines et des oxydes de phospholidine.

9. Utilisation de carbodiimides et/ou de polycarbodiimides oligomériques, répondant à la formule (I), à titre de stabilisateurs contre la dégradation hydrolitique de produits de polycondensation ou de polyaddition, contenant des radicaux ester.

10. Utilisation de carbodiimides et/ou de polycarbodiimides oligomériques, répondant à la formule (I), à titre de stabilisateurs contre la dégradation hydrolitique de polyuréthannes.
